# EUROPEAN PATENT SPECIFICATION

(11) **EP 0 695 183 B1**
(45) Date of publication and mention of the grant of the patent: **23.07.2003**
(21) Application number: 94913693.1
(22) Date of filing: 25.04.1994
(51) Int. Cl.: A61K 31/42, A61K 31/425, A61K 31/44, A61K 31/505

(54) **USE OF THIAZOLIDINEDIONES FOR THE TREATMENT OF ATHEROSCLEROSIS AND EATING DISORDERS**
VERWENDUNG VON THIAZOLIDINDIONEN ZUR BEHANDLUNG VON ATHEROSKLEROSE UND ESSSTÖRUNGEN
UTILISATION DE THIAZOLIDINEDIONES POUR LE TRAITEMENT DE L'ATHEROSCLEROSE ET DES DESORDRES DE L'ALIMENTATION

(30) Priority: 23.04.1993 GB 9308487
(43) Date of publication of application: 07.02.1996
(60) Divisional application: 03075243.0
(73) Proprietor: SmithKline Beecham plc, Brentford, Middlesex TW8 9GS (GB)
(72) Inventor: CAWTHORNE, Michael Anthony, Yew Tree Bottom Rd Epsom Surrey KT18 5XQ (GB); HINDLEY, Richard Mark, Yew Tree Bottom Rd Epsom Surrey KT18 5XQ (GB)
(74) Representative: Rutter, Keith, Dr.
(86) International application number: GB9400879
(87) International publication number: WO94025026

(56) References cited:
- EP-A- 0 306 228
- EP-A- 0 419 035
- WO-A-93/02079
- WO-A-94/05659
- 'The Merck Manual of Diagnosis and Therapy', 1992 see page 409

## Description

This invention relates to novel use of certain substituted thiazolidinedione derivatives.

European Patent Applications, Publication Numbers 0008203, 0139421,
0155845, 0177353, 0193256, 0207581 and 0208420 relate to thiazolidinedione derivatives which are disclosed as having hypoglycaemic and hypolipidaemic activity. Chem. Pharm. Bull 30 (10) 3580-3600 also relates to certain thiazolidinedione derivatives having hypoglycaemic and hypolipidaemic activities.

European Patent Application, Publication Number 0306228 discloses certain substituted thiazolidinedione derivatives of formula (A): 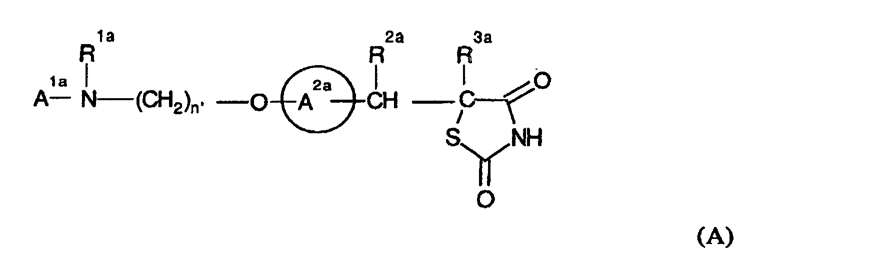 or a tautomeric form thereof and/or a pharmaceutically acceptable salt thereof, and/or a pharmaceutically acceptable solvate thereof, wherein:
A^{1a} represents a substituted or unsubstituted aromatic heterocyclyl group;
R^{1a} represents a hydrogen atom, an alkyl group, an acyl group, an aralkyl group,
   wherein the aryl moiety may be substituted or unsubstituted, or a substituted or unsubstituted aryl group;

R^{2a} and R^{3a} each represent hydrogen, or R^{2a} and R^{3a} together represent a bond;
A^{2a} represents a benzene ring having in total up to five substituents; and
n'represents an integer in the range of from 2 to 6. Such compounds are disclosed *inter alia* as being useful for the treatment and/or prophylaxis of cardiovascular disease and certain eating disorders.

It has now surprisingly been discovered that these compounds are of particular use in the treatment and/or prophylaxis of atherosclerosis. In addition these compounds are particularly useful for the regulation of appetite and food intake in subjects suffering from disorders associated with under-eating, such as anorexia nervosa, and disorders associated with over-eating, such as obesity and anorexia bulimia.

Accordingly, the present invention provides the use of 5-[4-[2-(N-methyl-N-(2-pyridyl)amino)ethoxy]benzyl]thiazolidine-2,4-dione or a tautomeric form thereof and/or a pharmaceutically acceptable salt thereof other than a maleate salt and/or a pharmaceutically acceptable solvate thereof, other than a hydrate of a maleate salt for the manufacture of a medicament for the treatment and/or prophylaxis of atheroscterosis.

Suitable pharmaceutically acceptable salts include salts of the thiazolidinedione moiety, and, where appropriate, salts of carboxy groups.

Suitable pharmaceutically acceptable salts of the thiazolidinedione moiety include metal salts especially alkali metal salts such as the lithium, sodium and potassium salts.

Suitable pharmaceutically acceptable salts of carboxy groups include metal salts, such as for example aluminium, alkali metal salts such as sodium or potassium, alkaline earth metal salts such as calcium or magnesium and ammonium or substituted ammonium salts, for example those with lower alkylamines such as triethylamine, hydroxy alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-b-phenethylamine, dehydroabietylamine, N,N'-bisdehydroabietylamine, glucamine, N-methylglucamine or bases of the pyridine type such as pyridine, collidine or quinoline.

Suitable pharmaceutically acceptable solvates include hydrates.

The salts and/or solvates of the compounds of formula (I) may be prepared and isolated according to conventional procedures for example sodium salts may be prepared by using sodium methoxide in methanol.

Suitable pharmaceutically acceptable salts of the thiazolidinedione moiety include metal salts especially alkali metal salts such as the lithium, sodium and potassium salts.

Said compound is hereinafter referred to as 'the Compound'. The Compound or the tautomeric form thereof, and/or a pharmaceutically acceptable salt thereof, and/or a pharmaceutically acceptable
solvate thereof, may be prepared using the processes described in EP 0306228. The contents of EP 0306228 are incorporated herein by reference

As mentioned above the compounds of the invention are indicated as having useful therapeutic properties:

The Compound or a tautomeric form thereof and/or a pharmaceutically acceptable salt thereof and/or a pharmaceutically acceptable solvate thereof, may be administered per se or, preferably, as a pharmaceutical composition also comprising a pharmaceutically acceptable carrier.

As used herein the term 'pharmaceutically acceptable' embraces compounds, compositions and ingredients for both human and veterinary use: for example the term pharmaceutically acceptable salt' embraces a veterinarily acceptable salt.

The composition may, if desired, be in the form of a pack accompanied by written or printed instructions for use.

Usually the pharmaceutical compositions of the present invention will be adapted for oral administration, although compositions for administration by other routes, such as by injection and percutaneous absorption are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose will normally contain an amount of the active ingredient in the range of from 0.1 to 1000 mg, more usually 0.1 to 500 mg, and more especially 0.1 to 250 mg.

Conveniently, the active ingredient may be administered as a pharmaceutical composition hereinbefore defined, and this forms a particular aspect of the present invention.

In the above mentioned treatments the Compound or a tautomeric form thereof and/or a pharmaceutically acceptable salt thereof and/or a pharmaceutically acceptable solvate thereof, may be taken in doses, such as those described above, one to six times a day in a manner such that the total daily dose for a 70 kg adult will generally be in the range of from 0.1 to 6000 mg, and more usually about 1 to 1500 mg.

In the treatment and/or prophylaxis of non-human mammals, especially dogs, the active ingredient may be adminstered by mouth, usually once or twice a day and in an amount in the range of from about 0.025 mg/kg to 25 mg/kg, for example 0.1 mg/kg to 20 mg/kg.

The therapeutic activity of the Compound, a tautomeric form thereof, a pharmaceutically acceptable salt thereof or a pharmaceutically acceptable solvate thereof may be demonstrated using conventional test methods, for example anti-atherosclerotic activity may be demonstrated using methods disclosed in Journal of Clinical Investigations 1992, Vol 89, page 706-711.

## Claims

1. The use of 5-(4-[2-(N-methyl-N-(2-pyridyl)amino)ethoxy]benzyl)-2,4-thiazolidinedione or a tautomeric form thereof and/or a pharmaceutically acceptable salt thereof, other than a maleate salt, and/or a pharmaceutically acceptable solvate thereof, other than a hydrate of a maleate salt, for the manufacture of a medicament for the treatment and/or prophylaxis of atherosclerosis.

## Patentansprüche

1. Verwendung von 5-(4-[2-(N-Methyl-N-(2-pyridyl)amino)ethoxy]benzyl)-2,4-thiazolidindion oder einer tautomeren Form davon und/oder eines pharmazeutisch verträglichen Salzes davon, welches von einem Maleatsalz verschieden ist, und/oder eines pharmazeutisch verträglichen Solvats davon, welches von einem Hydrat eines Maleatsalzes verschieden ist, zur Herstellung eines Medikaments zur Behandlung und/oder Prophylaxe von Atherosklerose.

## Revendications

1. Utilisation de 5-(4-[2-(N-méthyl-N-(2-pyridyl)-amino)éthoxy]benzyl)-2,4-thiazolidinedione ou d'une de ses formes tautomères et/ou d'un de ses sels pharmaceutiquement acceptables, autre qu'un maléate, et/ou d'un de ses produits de solvatation pharmaceutiquement acceptables, autre qu'un hydrate d'un maléate, pour la production d'un médicament destiné au traitement et/ou à la prophylaxie de l'athérosclérose.
